(19) Europäisches Patentamt
European Patent Office
Office européen des brevets

(11) EP 0 558 661 B1

(12) EUROPEAN PATENT SPECIFICATION

(45) Date of publication and mention of the grant of the patent:
**23.10.1996 Bulletin 1996/43**

(21) Application number: **92901960.2**

(22) Date of filing: **12.11.1991**

(51) Int Cl.[6]: **C07F 9/6515**, A61K 49/00, C07F 9/6524

(86) International application number:
**PCT/US91/08438**

(87) International publication number:
**WO 92/08725 (29.05.1992 Gazette 1992/12)**

# (54) POLYAZAMACROCYCLIC COMPOUNDS FOR COMPLEXATION OF METAL IONS

POLYAZAMAKROCYCLISCHE VERBINDUNGEN FÜR KOMPLEXIERUNG METAL-IONEN

COMPOSES POLYAZAMACROCYCLIQUES POUR LA COMPLEXATION DES IONS METAL

(84) Designated Contracting States:
**AT BE CH DE DK ES FR GB GR IT LI LU NL SE**

(30) Priority: **19.11.1990 US 615619**

(43) Date of publication of application:
**08.09.1993 Bulletin 1993/36**

(73) Proprietor: **BOARD OF REGENTS THE UNIVERSITY OF TEXAS SYSTEM**
**Austin, Texas 78701 (US)**

(72) Inventors:
- **SHERRY, A., Dean**
  **Dallas, TX 75248 (US)**
- **LAZAR, Istvan**
  **Richardson, TX 75080 (US)**
- **RAMASAMY, Ravichandran**
  **Dallas, TX 74252 (US)**
- **BRUCHER, Erno**
  **H-4032 Debrecen (HU)**

(74) Representative: **Allard, Susan Joyce et al**
**BOULT, WADE & TENNANT**
**27 Furnival Street**
**London EC4A 1PQ (GB)**

(56) References cited:
**EP-A- 0 382 582** **EP-A- 0 404 605**

- **JOURNAL OF THE CHEMICAL SOCIETY, CHEMICAL COMMUNICATIONS, no. 18, 1991, Cambridge (GB(; I. LAZAR, pp. 1252-1253**
- **MAGNETIC RESONANCE IN MEDICINE, vol. 30, December 1993; C.F.G.C. GERALDES et al., pp. 696-703**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 558 661 B1

Printed by Jouve, 75001 PARIS (FR)

## Description

The present invention relates to a series of new phosphorous containing macrocyclic chelates which bind certain biological ions with relative specificity. The $^{31}p$ resonance of these chelators shift to a new position in the NMR spectrum when a metal ion occupies the cavity of the chelate and the NMR chemical shift of the bound chelate differs for each metal ion. This property should be useful for monitoring the intracellular concentrations of certain biological cations including for example, $Mg^{2+}$, $Ca^{2+}$, and $Zn^{2+}$, by $^{31}P$ NMR. There are several fluorescent chelators commercially available for measuring intracellular cation concentrations and 1 or 2 fluorine containing chelators for NMR purposes. The fluorescent chelators will likely not be applicable for human studies and the $^{19}F$ chelators are not as likely to be applied clinically as the $^{31}P$ chelators of the present invention. Also, certain of the chelators when bound to $Gd^{3+}$ has properties which should make it a safe, effective contrast agent for magnetic resonance imaging.

A great number of biological systems require the diamagnetic cations, $Ca^{2+}$, $Mg^{2+}$, and $Zn^{2+}$ to regulate various reactions. The role of $Ca^{2+}$ as an intracellular messenger ion in many types of cells is well established [1]. $Mg^{2+}$ is a required cofactor for virtually all biological reactions involving ATP and may play an extensive role in the buffering of a greater variety of biological reactions [2]. $Zn^{2+}$ is bound rather tightly in the active sites of several enzymes and its role appears to involve polarization of chemical bonds to aid bond hydrolysis, oxidation-reduction, or group transfer reactions. However, free $Zn^{2+}$ may play a much wider role in some cells such as brain cells where it is known to be stored in neurons as storage granules and is mobilized during electrophysiologic activation [3].

An evaluation of the role of divalent cations in cell function has been limited by the availability of direct methods for measuring free cation concentration in cells and perfused organs. Presently available methods for measurement of divalent cations include indirect calculations based on equilibrium reactions [4], ion-selective micro-electrodes [5,6], and null point measurements using metallochromic dyes that are either microinjected into cells [7] or placed into the extracellular space with subsequent lysis of cells [8,9]. Virtually all of these methods are invasive in nature and require sample destruction prior to analysis. By contrast, NMR has made considerable advances as a non-invasive tool in measuring intracellular free divalent cation concentrations in perfused organs and intact cells.

Recently, fluorinated chelators have been effectively used to measure intracellular free $Ca^{2+}$ [10,11] and $Mg^{2+}$ [12,13] in cells and perfused organs by $^{19}F$ NMR. These chelators work reasonably well in isolated cell systems but suffer from unexpected line broadening when used in perfused organs [13,14]. Another disadvantage with these systems is the synthetic routes to these compounds limits the possible chelate structures and hence metal-ion selectively that may be designed into the chelate.

US-A-4639365 describes the chelates of gadolinium with 1,4,7-triazacyclononane-N,N',N"-triacetate, 1,4,7,10-tetracylododecane-N,N",N", N"'-tetraacetate and 1,5,9-triazacylodecane-N,N',N"-triacetate as useful as NMR contrast agents.

EP-A-0382582 describes tetra-azomacrocycles based on 1,4,7,10-tetracyclododecane in which at least one nitrogen atom has a group $-(CH_2)_q$ $-P(O)(XH)R^4$ attached thereto, where X is oxygen or sulfur, $R^4$ is hydrogen, alkyl or alkoxy and q is 0, or an integer of from 1 to 6.

Aspects of the present invention include the synthesis and development of a series of triaza and tetraaza macrocyclic phosphonate monoesters as $^{31}P$ NMR indicators to detect intracellular free cations in biological systems.

The present invention also relates to NMR imaging of living subjects, sometimes referred to as MRI (magnetic resonance imaging). More specifically, it relates to agents which can be used to enhance NMR contrast in such subjects.

Nuclear magnetic resonance (NMR) has been used for many years as a means of chemical analysis. NMR is a type of radio frequency spectroscopy which is based upon small energy differences between electrically charged atomic nuclei which are spinning parallel or antiparallel to an applied magnetic field. When radio frequency energy is applied to the sample, these spining atomic nuclei change spin states and in doing so, absorb some of the radio frequency energy. Nuclei in slightly different chemical environments within the same molecule change spin state at slightly different energies and this produces characteristic absorptions or resonances which help identify the molecular structure.

NMR has more recently been used in examinations of the human body. Other methods such as computerized axial tomography (CAT scanning) have been used in the past for this purpose, and still are. However, because NMR does not use ionizing radiation, it is believed to have some safety advantages over CAT. Thus, NMR is an advantageous method of producing cross-sectional images of the human body.

The quality of the images obtained from an NMR scan is based on two properties: the proton densities of the various tissues and differences in proton relaxation rates. The proton density of tissues cannot be readily altered. Proton relaxation rates can be adjusted by adding a paramagnetic relaxation agent, more commonly known as a "contrast agent." Contrast agents enhance the contrast in NMR images between magnetically similar but histologically dissimilar tissues.

Gadolinium has been tested as a contrast agent in the past because it has a large magnetic moment, which efficiently relaxes magnetic nuclei. Gadolinium's strong paramagnetic properties are the result of its seven unpaired electrons.

One drawback of gadolinium as a contrast agent is its toxicity to animals. One possible remedy for this problem is to incorporate gadolinium in a compound that would pass through the body and be excreted without releasing toxic gadolinium ions. Unfortunately, the rare earth elements, such as gadolinium, do not form stable covalent bonds with organic molecules, so such molecules can decompose *in vivo* and release the toxic ions.

There is a need for effective contrast agents which avoid the toxicity problems inherent in using gadolinium. Further, there is a need for new and better contrast agents, whether they include gadolinium or another paramagnetic metal.

The present invention relates to a new polyazamacrocyclic compound or a salt thereof and its uses. The compound having the formula:

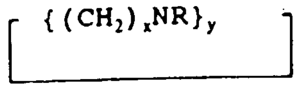

where

x is 2, 3 or a combination of p 2(s) and q 3(s) where p + q = y;
y is 3 or 4;
R is $CH_2)_zP(=O)OR^1R^2$;
when y is 3, $R^1$ is 0-alkyl and $R^2$ is attached to the oxygen atom and is alkyl or

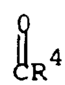

where $R^4$ is alkyl, cycloalkyl or aryl; and
z is 1 to 3;
when y is 4, $R^1$ is $OR^3$ where $R^3$ is alkyl with 2 or more carbon atoms and $R^2$ is attached to the oxygen atom and is hydrogen, alkyl or

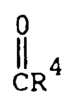

where $R^4$ is alkyl, cycloalkyl or aryl; and
z is 1 to 3.

A preferable alkyl is $C_nH_{1+2n}$ where n is 1-20; a preferable cycloalkyl is $C_nH_{2m-1}$ when m is 1-20; and a preferable aryl is phenyl.

In one important embodiment, this compound may be complexed with a metal to be a polyazamacrocyclic compound-metal complex having the formula

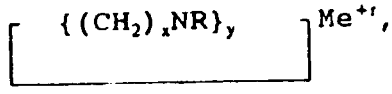

where

x is 2, 3 or a combination of p 2(s) and q 3(s) where p + q = y;
y is 3 or 4;
R is $CH_2)_zP(=O)OR^1R^2$;
when y is 3, $R^1$ is 0-alkyl and $R^2$ is attached to the oxygen atom and is alkyl or

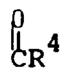

where $R^4$ is alkyl, cycloalkyl or aryl; and
z is 1 to 3; and
when y is 4, $R^1$ is $OR^3$ where
$R^3$ is alkyl with 2 or more carbon atoms and $R^2$ is attached to the oxygen atom and is hydrogen, alkyl or

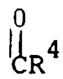

where $R^4$ is alkyl, cycloalkyl or aryl; and
z is 1 to 3;
r is 2 or 3; and
Me is a metal ion.

The y designation characterizes the compound as triazamacrocyclic or tetraazamacrocyclic. The x is preferably 2, although 3 is feasible under many circumstances. Combinations of p 2(s) and q 3(s) for x are of course readily produced but the total of p + q must be y for the number of units in the polyaza macrocycle.

In a preferred embodiment of either the compound or its metal complex y is 3, p is 1 and q is 2 or p is 2 and q is 1.

In another preferred embodiment of the compound or its metal complex, y is 4, p is 1 and q is 3, p is 2 and q is 2 or p is 3 and q is 1 and z is most preferably 1. n is preferably 2.

The $Me^{+r}$ is preferably $Ca^{+2}$, $Mg^{+2}$, $Zn^{+2}$ or $Gd^{+3}$. Other characteristic divalent or trivalent metal ions, particularly paramagnetic lanthanides may also be so complexed.

The present invention comprises a method for assessing intracellular concentration of one or more divalent metal ions. The method comprising treating cells, under conditions facilitating intracellular localization of a polyazamacrocyclic compound or a salt thereof, the compound having the formula

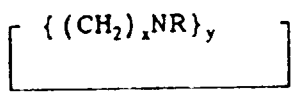

where

x is 2, 3 or a combination of p 2(s) and q 3(s) where p + q = y;
y is 3 or 4;
R is $CH_2)_zP(=O)OR^1R^2$;
when y is 3, $R^1$ is 0 alkyl and $R^2$ is attached to the oxygen atom and is alkyl or

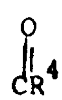

where $R^4$ is alkyl, cycloalkyl or aryl; and
z is 1 to 3 and
when y is 4, $R^1$ is $OR^3$ where $R^3$ is alkyl with 2 or more carbon atoms and $R^2$ is attached to the oxygen atom and is hydrogen, alkyl or

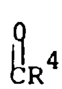

where $R^4$ is alkyl, cycloalkyl or aryl; and

z is 1 to 3;
and measuring shifts in the $^{31}P$ NMR spectrum, said shifts being proportional to intracellular divalent metal concentration. In one embodiment cells are treated with the acid anhydride form of the compound of the present invention
(*i.e.* where $R^2$ is

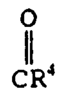 )

which passes into the cells, is there hydrolyzed to the acid form (*i.e.* where $R^2$ is H) and complexes with intracellular metal ions. The metals currently most amenable to such measurement are calcium, magnesium or zinc, magnesium being preferred.

In one important application, the present invention involves the use of a polyazamacrocyclic compound-metal complex having the formula:

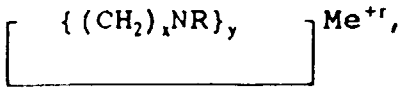

where

x is 2, 3 or a combination of p 2(s) and q 3(s) where p + q = y;
y is 3 or 4;
R is $(CH_2)_zP(=O)OR^1R^2$;
when y is 3, $R^1$ is 0 alkyl and $R^2$ is attached to the oxygen atom and is alkyl or

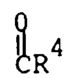

where $R^4$ is alkyl, cycloalkyl or aryl; and
z is 1 to 3;
when y is 4, $R^1$ is $OR^3$ where
$R^3$ is alkyl with 2 or more carbon atoms and $R^2$ is attached to the oxygen atom an is hydrogen, alkyl or

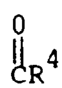

where $R^4$ is alkyl, cycloalkyl or aryl; and
z is 1 to 3;
Me is a paramagnetic metal; and
r is 2 or 3;

as a magnetic resonance image enhancement agent. The metal is preferably gadolinium and r is 3.

Figure 1 schematically illustrates the structure of NOTPME (where R is $CH_2CH_3$ and $R^1$ is H).

Figure 2 describes potentiometric titration curves of 2 mM NOTPME (a) and those obtained in the presence of equivalent amounts of $Ca^{2+}$ (b), $Mg^{2+}$ (c), and $Zn^{2+}$ (d).

Figure 3 shows $^{31}P$ NMR spectra of 5 mM NOTPME as a function of added (total) magnesium. $Mg^{2+}$ concentrations from bottom to top are 0, 0.5, 1.5, 3 mM respectively. Sample constituents and spectral parameters are described in

methods section.

Figure 4 shows $^{31}P$ NMR spectra of 5mM NOTPME in the presence of (A) 2.05 mM $ZnCl_2$ , and (B) 38.5 mM $CaCl_2$. Sample composition and spectral parameters are given in methods section.

Figure 5 describes pH dependence of the $^{31}P$ NMR chemical shifts of 5 mM NOTPME (a), $Ca(NOTPME)^-$ (b),Zn $(NOTPME)^-$ (c), and $Mg(NOTPME)^-$ (d).

Figure 6 shows $^{31}P$ NMR spectra of (a) normal RBCs and (b) NOTPME loaded RBCs.

Figure 7 schematically illustrates the structure of DOTEP.

Figure 8 shows the $^{31}P$ NMR spectrum of DOTEP and a DOTEP-calcium complex.

Figure 9 describes the decomposition rate of gadolinium DOTA and gadolinium-DOTEP complexes in 0.1N HCl.

Figure 10 schematically shows the compound 1,5,9-triazacyclododecane.

Figure 11 schematically shows the compound 1,4,8,12-tetracyclopentadecane.

Figure 12 schematically shows the compound 1,4,8,11-tetraazacyclotetradecane.

**EXAMPLE 1**

__Triazamacrocyclic Compounds__

1,4,7-Triazacyclononane-N,N',N"-tris(methylenephosphonate monoethylester) (NOTPME) has been synthesized, characterized and analyzed for use as a $^{31}P$ NMR indicator of intracellular $Mg^{2+}$ and $Zn^{2+}$ ions. The $^{31}P$ NMR spectrum of this chelate in the presence of metal ions shows characteristic resonances for the free chelate, $Mg(NOTPME)^-$, and $Zn(NOTPME)^-$ and $Ca(NOTPME)^-$. Stability constants for NOTPME complexes with $Ca^{2+}$ and $Mg^{2+}$ have been obtained by potentiometry and NMR and its complex with $Zn^{2+}$ by potentiometry. This chelate has a 10 fold higher affinity for $Mg^{2+}$ than for $Ca^{2+}$ at physiological pH values. Its affinity for $Zn^{2+}$ is so high that accurate binding constants could not be determined by NMR. In the presence of $Mg^{2+}$, NOTPME is readily loaded into red blood cells. $^{31}P$ chemical shifts of the free chelate and its metal complexes are far downfield from the typical phosphorous containing metabolites observed in biological systems, thus making it possible to monitor intracellular cation concentration and cell energetics simultaneously.

__Materials__

1,4,7-triazacyclononane, paraformaldehyde, diethyiphosphite, and activated carbon Darco G-60 were purchased from Aldrich Chemical Company. $MgSO_4$ was from Mallickrodt, sodium hydroxide, and benzene from J. T. Baker, and diethylether from Fisher Scientific. All chemicals were of highest purity and were used without further purification. Solutions of $ZnCl_2$, $CdCl_2$, $MgCl_2$ and Ca $Cl_2$ were standardized complexometrically.

__Synthesis of NOTPME__

1,4,7-Triazacyclononane (1.91 g, 14.71 mmol) and diethylphosphite (7.018 g, 16.94 mmol, 15% excess) were dissolved in 125 ml of benzene and heated to reflux. Anhydrous paraformaldehyde (1.727 g, 30% excess) was added in small portions to the above refluxing mixture while the benzene-water azeotropic mixture was removed by distillation. After the addition of paraformaldehyde was complete, the entire solution was boiled for 30 minutes and then evaporated to obtain a yellow viscous oil. The oil was dissolved in 150 ml anhydrous diethylether and dried with anhydrous $MgSO_4$ overnight. $MgSO_4$, along with a white precipitate which formed, were filtered off and discarded. The filtrate was decolorized with activated carbon and filtered. The filtrate was evaporated in vacuum to obtain a viscous oil of 1,4,7-triazacyclononane-N,N',N"-tris(methylenephosphonate diethylester) (NOTPDE). Pure NOTPDE was obtained in 96% yield (9.21 g, 14.17 mmol) and was used for the synthesis of NOTPME (structure shown in Figure 1) without further purification. $^1H$ NMR data of NOTPDE in $CDCl_3$ (TMS at zero) are as follows: $\delta$ (ppm) : 1.33 (t, 18H, $-CH_3$), 2.97 (s, 12H, $N-CH_2$), 3.00 (d, 6H, $P-CH_2$), 4.13 (p, 12H, $O-CH_2$).

9.20 g of NOTPDE (14.15 mmol) was mixed with 2.50 g of NaOH in 9 ml $H_2O$) and after 2 hours the entire reaction mixture was boiled until a clear solution was obtained (approximately 5 minutes). The solution was cooled to room temperature and was allowed to stand overnight. The crystals formed were filtered off from the viscous mother liquor using a pressure filter funnel with a coarse porosity grade filter disc. The crystals were washed once with cold absolute ethanol, three times with absolute ethanol-diethylether (1:1) mixture and finally with diethyl ether. The crystals of $Na_3$NOTPME were dried in dry nitrogen stream at 25°C for 2 hours. Traces of $H_2O$ and ethanol were removed upon vacuum drying (10 mm Hg) NOTPME for 5 hours at 50°C. Pure NOTPME thus obtained were white crystals, very hygroscopic, readily soluble in $H_2O$, and fairly soluble in chloroform. The yield of pure NOTPME was 40.8% (3.24 g, 5.77 mmol).

$^1H$ NMR ($D_2O$, HDO peak set as reference at 4.90 ppm), $\delta$ (ppm): 1.23 (t, 9H, $-CH_3$), 2.54 (s, broad, 6H, $P-CH_2$),

2.79 (s, broad, 12 H, N-$CH_2$), 3.91 (p, 6H, O-$CH_2$).

NMR Measurements

$^1H$ NMR data was obtained on JEOL FX-200 NMR spectrometer using a 10 mm probe operating at room temperature. Studies on solutions of NOTPME and its complexes were performed on a General Electric GN-500 NMR spectrometer. A 10 mm broad band probe was tuned to 202.4 MHz for $^{31}P$ detection. Shifts for NOTPME and its complexes were measured using 85% $H_3PO_4$ as an external standard. Probe temperatures were accurate to ± 0.5°C.

Samples of NOTPME used for $K_D$ determinations by NMR consisted of 115 mM KCl, 20 mM NaCl and 10 mM HEPES buffered with Tris base at pH 7.4. Varying concentrations (typically 0.5 to 10 mM) of $Mg^{2+}$, $Ca^{2+}$ or $Zn^{2+}$ were added to the sample and the resulting $^{31}P$ NMR spectrum obtained. Resonance areas were determined by integration of peaks using the GE software.

Potentiometric Measurements

Potentiometric titrations were conducted at 25°C using a Corning Ion Analyzer (model 250) and a Metrohm Dorsimat automatic burette (Brinkman Instruments). The hydrogen ion concentration was obtained from the measured pH values by the method suggested by Irving *et al.* [15]. $Na_3$NOTPME was dissolved in 0.1 M tetramethylammonium chloride, the pH adjusted to low pH value with HCl and titrated with 0.098 M KOH. KOH was standardized by potentiometric titration against potassium hydrogen phthalate and stored under $N_2$ atmosphere. The hydrogen ion activity coefficient and $K_w$ were determined separately in these same salt solutions. Stability constants of $Zn(NOTPME)^-$, $Mg(NOTPME)^-$, and $Ca(NOTPME)^-$ were determined by potentiometric titration of solutions containing 1:1 ratio of metal and ligand. In all of the titrations, samples were covered by a layer of cyclohexane to exclude $CO_2$.

Protonation constants ($K_{HiL}$) and stability constants ($K_{ML}$, and $K_{MHL}$) are defined by the following equations:

$$K_{HiL} = [H_iL] / \{[H_{i-1}L] \{H^+]) \qquad (1)$$

$$K_{ML} = [ML] / \{[M] [L]) \qquad (2)$$

$$K_{MHL} = [MHL] / \{[ML] [H^+]\} \qquad (3)$$

Protonation and stability constants were obtained from the potentiometric data using a simplex non-linear algorithm [16] run on an IBM PC.

Red Blood Cell Loading of NOTPME

Fresh whole blood was obtained placed in heparinized tubes and centrifuged at 3000 g for 6 min. to remove the buffy coat. RBCs were then washed three times in 5 mM phosphate buffered saline at pH 7.4. RBCs at 50% hematocrit were suspended in the loading medium containing 120 mM NaCl, 5 mM $MgCl_2$, 10 mM HEPES pH 7.4, 10 mM glucose, and 10 mM NOTPME and incubated at 37°C for 12 hours. No lysis of RBCs were observed during the loading procedure. The RBCs were centrifuged and the supernatant discarded. The RBCs were washed twice with phosphate buffered saline at pH 7.4 before suspension in an isotonic medium.

Protonation Studies

A representative potentiometric titration curve for NOTPME in 0.1 M tetramethylammonium chloride and 25°C is illustrated in Figure 2. Three protonation constants ($pK_1$ = 11.8, $pK_2$ = 3.65, and $pK_3$ = 1.4) were obtained from these data. The $^{31}P$ NMR spectrum of NOTPME was also measured as a function of pH (data not shown) and the protonation constants obtained from these data were in general agreement with those obtained by potentiometry. The phosphorous shifts are very sensitive to protonation of the nitrogens in the polyaza ring, similar to that observed for the parent phosphonate NOTP [17]. The first two protonations (log K = 11.8, 3.65) result in $^{31}P$ shifts to low frequency which is consistent with protonation at two ring nitrogens [17]. This indicates that the first nitrogen protonations in NOTP versus NOTPME are quite similar (log $K_1$ = 12.1 versus 11.8, respectively) while the second nitrogen protonations are dramatically different (log $K_2$ = 9.4 versus 3.65). These differences in $pK_2$ may reflect differences in the ability of the

phosphonate versus the phosphonate monoester side chains to form internal hydrogen bonds with the protonated nitrogens. This would be consistent with the greater basicity of the phosphonate oxygens in NOTP (protonation constants between 6.0 and 7.5) over the phosphonate ester oxygens in NOTPME (protonations below 1.4). $^{31}P$ NMR spectra of NOTPME exhibited a single resonance over the entire pH range, indicating rapid exchange between all protonated species.

Complexation Studies

Figure 2 also shows potentiometric titration data for NOTPME in the presence of one equivalent of $Mg^{2+}$, $Ca^{2+}$, or $Zn^{2+}$. The stability constants for $Mg(NOTPME)^-$, $Ca(NOTPME)^-$, and $Zn(NOTPME)^-$ derived from these data are summarized in Table I.

TABLE I

| Stability constants of the NOTPME complexes with $Ca^{2+}$, $Mg^{2+}$, and $Zn^{2+}$. | | | |
|---|---|---|---|
| Metal ion | $K_D$ (at 25°X) | $K_D$ (at 37°C) | log $K_{ML}$ |
| $Ca^{2+}$ | 50 mM | 47.62 mM | 5.1 |
| $Mg^{2+}$ | 5.77 mM | 4.66 mM | 6.3 |
| $Zn^{2+}$ | $(10^{-11}$ M$)^a$ | _ | 15.4 |
| $K_D$ values were obtained from NMR data while log K values were determined by potentiometry at 25°C. | | | |

$^a K_D$ estimated from the thermodynamic value (log $K_{ML}$ = 15.4) by considering the ligand pK values and the proton concentration at pH 7.4.

$Zn^{2+}$ forms a considerably more stable complex with NOTPME than either $Mg^{2+}$ or $Ca^{2+}$. This largely reflects the propensity of $Zn^{2+}$ to form stronger $M^{2+}$-N bonds than more ionic species, $Mg^{2+}$ and $Ca^{2+}$. Both $Mg(NOTPME)^-$ and $Ca(NOTPME)^-$ are less stable than their respective NOTP complexes [18], which again reflects the more acidic nature of phosphonate ester side-chain ligands. However, NOTPME, like NOTP forms more stable complexes with the smaller $Mg^{2+}$ ion than with $Ca^{2+}$. This reflects the size selectivity of the triazacyclononane macrocycle in both cases.

Figure 3 displays the $^{31}P$ NMR spectrum of a solution containing 5 mM NOTPME as a function of added $Mg^{2+}$. The spectra show that $Mg^{2+}$ bound NOTPME is in slow exchange with "free" NOTPME. Figure 4 shows $^{31}P$ NMR spectra of 5 mM NOTPME in the presence of 2.05 mM $Zn^{2+}$ and 38.5 mM $Ca^{2+}$. As expected based upon the potentiometrically determined stability constants, there is more Zn(NOTPME) present in this solution than in an equivalent solution containing $Mg^{2+}$. Interestingly, the chemical shifts of $Mg^{2+}$ and $Zn^{2+}$ bound NOTPME are quite different and, indeed, it is possible to obtain characteristic resonances for Zn(NOTPME) and Mg(NOTPME) in a mixture containing both metal ions. $Ca^{2+}$, as expected, binds much more weakly and a resonance characteristic of $Ca(NOTPME)^-$ only becomes visible after 32 mM $Ca^{2+}$ has been added. The chemical shift of $Ca(NOTPME)^-$ (22.1 ppm) was also different form those of $Mg(NOTPME)^-$ (23.5 ppm) and $Zn(NOTPME)^-$ (23.1 ppm). This probably reflects the larger size of $Ca^{2+}$ versus $Mg^{2+}$ or $Zn^{2+}$ and its inability to fit into the cyclononane cavity.

The area of resonances corresponding to free and metal bound NOTPME were obtained by integration and their concentrations estimated. The $K_D$ values obtained at 25°C by NMR for $Mg^{2+}$ and $Ca^{2+}$ complexes are 5.77 mM and 50 mM, respectively. In comparison, the fluorinated chelator, MF-APTRA, reported by others [12] has a $K_D$ of 1 mM for $Mg^{2+}$ and 12 μM for $Ca^{2+}$.

Table I also shows the $K_D$ for $Mg(NOTPME)^-$, and $Ca(NOTPME)^-$ obtained by NMR at 37°C. Clearly, the magnitude of differences in stabilities between $Mg(NOTPME)^-$, and $Ca(NOTPME)^-$ at 37°C remain the same. This reconfirms the higher affinity of NOTPME for $Mg^{2+}$ over $Ca^{2+}$ at physiological temperature. It was not possible to obtain $K_D$ values for $Zn(NOTPME)^-$ from the NMR data because the titrated $Zn^{2+}$ was completely bound to NOTPME under virtually all measurable conditions.

Figure 5 shows the $^{31}P$ NMR chemical shifts of the NOTPME complexes of $Ca^{2+}$, $Mg^{2+}$, and $Zn^{2+}$ as a function of pH between 6 and 10. In solutions containing 1:1 metal ion and ligand, the resonance characteristic of $Zn(NOTPME)^-$ is visible over this entire pH range, that characteristic of $Mg(NOTPME)^-$ is detectable only above pH 6.4, while that characteristic of $Ca(NOTPME)^-$ is only visible above pH 7.8. These differences clearly demonstrate the greater selectivity of NOTPME for $Mg^{2+}$ over $Ca^{2+}$ at physiological pH (ca. 7.4). As shown, chemical shifts of the metal bound species are all virtually independent of pH over this range.

Determination of Intracellular Free $Mg^{2+}$ Concentration in Human Erythrocytes

Figure 6 illustrates one application of NOTPME to detect intracellular free $Mg^{2+}$ in RBCs. NOTPME loaded RBCs (Figure 6.b) were prepared by incubating RBCs at 37°C in a loading medium containing NOTPME in the presence of

5mM $MgCl_2$ for 12 hours. NOTPME loading into RBCs was not observed in the absence of $MgCl_2$ in the loading medium. Control RBCs are shown in Figure 6a.

As in the *in vitro* studies, two resonances were observed corresponding to free and $Mg^{2+}$ bound NOTPME in RBCs. The intracellular free $Mg^{2+}$ concentration in RBCs was estimated using the following equation:

$$[Mg^{2+}] = K_D\ [MgNOTPME] / [NOTPME]$$

An intracellular free $Mg^{2+}$ concentration of 1.71 mM was obtained in this particular example. Since intracellular free $Mg^{2+}$ levels in RBCs normally range between 0.2 - 0.3 mM [12,19,20], the high concentration of intracellular free $Mg^{2+}$ observed in this experiment must have resulted from transport of $Mg^{2+}$ as a complex with NOTPME. Upon addition of the divalent cation ionophore A23187 to the suspension medium, an increase in intracellular free $Mg^{2+}$ concentration (to 1.90 mM) was observed (data not shown). These results show that NOTPME can easily detect small changes in free $Mg^{2+}$ levels in intact cells.

The complex forming characteristics of NOTPME are strongly influenced by the very high value of the first protonation constant as well as by the conformational and size requirements of the triaza ring. The fact that $Zn^{2+}$, $Ca^{2+}$, and $Mg^{2+}$ complexes of NOTPME could be studied by usual potentiometric method indicates that the complexes are formed relatively quickly in solution. As seen in Table I, $Zn^{2+}$ forms a more stable complex with NOTPME than does either $Mg^{2+}$ or $Ca^{2+}$. This same trend was observed for the triazacyclononane triacetate chelate, NOTA, with these same ions; reported log K values for $Zn(NOTA)^-$, $Mg(NOTA)^-$, and $Ca(NOTA)^-$ are 18.3, 9.69, and 8.92, respectively [21]. Thus, the carboxylate chelate forms more stable complexes with all three metal ions perhaps due to the greater basicity of the carboxylate oxygen ligands over the phosphonate oxygen ligands. The parent phosphonate, NOTP, shows the same trend in its metal complexing ability with $Zn^{2+}$, $Mg^{2+}$, and $Ca^{2+}$ but in this case the stability constants of the resulting complexes are all several orders of magnitude higher than the corresponding values with NOTPME or NOTA. The reported log K values for $Zn(NOTP)^{4-}$, $Mg(NOTP)^{4-}$, and $Ca(NOTP)^{4-}$ are 24.9, 11.0, 6.4 respectively [18]. Since the nitrogens and the phosphonate oxygens in NOTP are considerably more basic than those in NOTPME, the resulting stability constants with these metal ions are all larger. The trend $Zn^{2+} >> Mg^{2+} > Ca^{2+}$, however, is preserved.

NOTPME has several advantages over the fluorinated chelators such as BAPTA and APTRA for measurement of intracellular $Mg^{2+}$ in biological systems. These include (a) ease of synthesis, (b) higher affinity for $Mg^{2+}$ than for $Ca^{2+}$, (c) no significant exchange contribution to the bound and free ligand resonances, and (d) the three magnetically equivalent $^{31}P$ nuclei in this chelate provides an attractive NMR nucleus for biological tissue since the cell energetics may be measured simultaneously. The significant selectivity for $Mg^{2+}$ over $Ca^{2+}$ exhibited by NOTPME at physiological pH is of great advantage for monitoring $Mg^{2+}$ in biological systems without concern about interference from $Ca^{2+}$. It is important to emphasize that the resonances of NOTPME and its magnesium complex do not overlap with the phosphorous containing metabolites in tissue. Thus NOTPME provides versatile approach of measuring changes in intracellular free magnesium as well as changes in metabolites during various physiological events by $^{31}P$ NMR. Although it is fortuitous that the $K_D$ $Mg(NOTPME)^-$ at physiological pH is in the range required for measurement of intracellular $Mg^{2+}$ in many biological systems, it may prove possible to fine tune the affinity of NOTPME for $Mg^{2+}$ by changing the alkyl substituents on the phosphonate oxygens or by adding an alkyl chain to the carbon which links the phosphonate groups to the triaza ring.

## EXAMPLE 2

### Tetraazamacrocyclic compounds DOTEP Synthesis

DOTEP, shown in Figure 7, was prepared as follows. 2 ml of dichloroethylphosphine was slowly mixed with ice to form the corresponding ethylphosphinic acid. After warming to room temperature, 390 mg of 1,4,7,10-tetraazacyclododecane tetrahydrochloride (cyclen.4HCl) (Parrish Chem. Co., Ogden, Utah) was added and the mixture heated to boiling under a nitrogen atmosphere. A solution containing 157 mg of paraformaldehyde dissolved in 10 ml of 6M HCl was added at a rate of 0.5 ml/hr, while the mixture continued to reflux. The final mixture was refluxed an additional 4 hours then cooled to room temperature. This solution was concentrated under vacuum to a viscous oil, redissolved into 6 ml of water and loaded onto a DOWEX 50Wx4 (hydrogen form) cation exchange column (7.5 ml bed volume). The column was washed to neutrality with water and the product eluted with 60 ml of 0.66 M HCl. The fractions containing DOTEP were combined, evaporated, redissolved in absolute ethanol and evaporated to a white solid. This solid was dispersed into anhydrous ether, filtered off, pre-dried under nitrogen and dried under vacuum at 60-70°C to yield a white, very hygroscopic solid (360 mg, 44% yield). This solid was stored in sealed ampoules. Elemental analysis and potentiometry shows the solid to be DOTEP.2HCl

**DOTEP APPLICATIONS**

A. Ca(DOTEP)$^-$ as a $^{31}$P NMR indicator of $[Ca^{2+}]_{free}$

The $^{31}$P spectrum shown in Figure 9 was obtained on a solution containing approximately 1 mM $Ca^{2+}$ and 3 mM DOTEP in an aqueous solution at physiological pH. Clearly, the $^{31}$P resonances of the free and bound forms of DOTEP have distinct chemical shifts and neither overlaps with the $^{31}$P resonances of metabolites normally observed in tissue. This indicates that when DOTEP is loaded into cells by making an appropriate hydrolysable ester, the DOTEP trapped in cells would give a direct measure of $[Ca^{2+}]_{free}$ using the simple relationship,

$$[Ca^{2+}]_{free} = K_D \cdot \frac{[Ca(DOTEP)]}{[DOTEP]_{free}}$$

where $K_D$ = 11 $\mu$M at pH 7.4

The value of $K_D$ at pH 7.4 reported above was determined by $^{31}$P NMR (measuring the free and bound integrals as a function of pH in solutions containing known amounts of $[Ca^{2+}]_{total}$ and $[DOTEP]_{total}$) and by pH potentiometry. This value of $K_D$ indicates that DOTEP will be useful for measuring $[Ca^{2+}]_{free}$ over the approximate range, 1-50 $\mu$M.

B. Gd(DOTEP)$^-$ as a MRI contrast agent.

DOTEP also forms stable complexes with $Gd^{3+}$ (log K approximately equal to 16). The resulting complex has a favorable water proton relaxivity (R = 5.1 $s^{-1}mM^{-1}$) and the complex has many of the same kinetic advantages as Gd (DOTA)$^-$, as shown by the data of Figure 10. In this experiment, Gd(DOTEP)$^-$ and Gd(DOTA)$^-$ were dissolved in 0.1M HCl and their decomposition to free $Gd^{3+}$ and free ligand were followed by water relaxation measurements. As shown, the half-lives for decomposition of the two complexes in strong acid were approximately 110 hrs. for Gd(DOTA)$^-$ versus 30 hrs. for Gd(DOTEP)$^-$. This indicates that the decomposition rates for both complexes at pH 7.4 will be several orders of magnitude longer than this (or about $10^6$ hrs.) and therefore should be quite safe for use in humans.

**EXAMPLE 3**

Polyazamacrocyclic N-alkyl phosphonate or phosphine acid anhydrides

**Synthesis of NOTPME-AC**

[NOTPME-AC = 1,4,7-triazacyclononane-N,N',N"-tris(methylenephosphonate monoethylester acetic acid anhydride)]

NOTPME trisodium salt (178.63 mg, 0.318 mmol) was dissolved in 4.0 ml anhydrous chloroform (freshly distilled from over $P_4O_{10}$) and 120 microliter acetyl chloride was added. The solution was stirred at room temperature for 24 hours in a tightly closed vessel. The solution was then evaporated under dry nitrogen atmosphere to a yellowish, very viscous oil. To the oil anhydrous ether was added and NOTPME-AC (precipitated as a sodium chloride adduct) was filtered off and dried at 35-40°C under dry nitrogen. NOTPME-AC (234.24 mg, 92.3% calculated on the basis of NOTPME-AC.3NaCl formula weight) is an extremely hygroscopic, water sensitive powder. It is readily soluble in water, chloroform and DMSO.

$^1$H NMR ($CDCl_3$/TMS): 1.31(t,9H,-$CH_3$), 2.24 & 2.21 (two sg(s), 9H, C(O)-$CH_3$), 3.29 (sg, v.br.,18H, N-$CH_2$-C and N-$CH_2$-P), 4.12 & 4.30 (two m(s), 6H, O-$CH_2$).

**Synthesis of NOTPME-B**

[NOTPME-B = 1,4,7-triazacyclononane-N,N',N"-tris(methylenephosphonate monoethylester benzoic acid anhydride]

NOTPME trisodium salt (164.82 mg, 0.294 mmol) and benzoyl chloride (105 microliter, 0.883 mmol) were reacted in 4.0 ml anhydrous chloroform at room temperature for 24 hours. The rection mixture was worked up with identical method than was used in the synthesis of NOTPME-AC. The product NOTPME-B was get as a sodium chloride adduct (204.32 mg, 70.7% calculated on the basis of NOTPME-B.3NaCl formula weight). NOTPME-B is a very hygroscopic, water sensitive powder. It is soluble in water and chloroform and DMSO.

$^1H$ NMR ($CDCl_3$/TMS): 1.29 (t,-$CH_3$) , 3.32 (m, V.br., N-$CH_2$-C and N-$CH_2$-P), 4.10 & 4.35 (two m(s), O-$CH_2$), 7.49 (m, Ar-H), 8.02(d,Ar-H).

It is understood to those of skill in the art that a variety of aryl halides may be used to prepare analogous phosphonate or phosphine acid anhydrides with only a minimum of efforts to define optimal synthetic procedures. Additionally, it is clear that facile leaving groups such as carbonyl-containing alkyls may be preferred for greater stability in an aqueous medium and yet still pass into cells for partial decomposition and use in NMR metal analyses.

Red Blood Cell Loading of NOTPME Derivatives

NOTPME derivatives used are the benzoyl and acetyl derivatives. Fresh whole blood was obtained in heparinized tubes and centrifuged at 3000g for 3 minutes to remove the buffy coat. Packed red blood cells are then washed three times in 5 mM phosphate buffered saline at pH 7.4. Red blood cells at 50% hematocrit were suspended in the NOTPME derivative containing loading medium. The loading medium contained 130 mM NaCl, 5 mM NOTPME derivative (benzoyl or acetyl derivative), 1 mM adenine, 10 mM glucose, and 10 mM HEPES at pH 7.4. Red cells in the above loading medium were incubated at 25°C for 1 hour. No lysis of red blood cells were observed during the loading procedure. After loading with NOTPME derivatives for one hour, the red cell suspension was centrifuged and the supernatant discarded. The red cells are washed twice with 5 mM phosphate buffered saline at pH 7.4 before suspension in an isotonic saline for NMR measurements. The $^{31}P$ NMR measurements indicated that these NOTPME derivatives get loaded inside red cells and get hydrolysed to NOTPME. After NMR measurements the sample was centrifuged and the supernatant analyzed for any leakage of NOTPME. No leak of NOTPME was observed during the time course of NMR measurements. thus, it is clear that these NOTPME derivatives enter the red cells, get hydrolyzed to NOTPME and remain inside red cells.

**EXAMPLE 4**

Prophetic Synthesis of Generic Polyazamacrocyclic N-Alkylphosphonates, Polyazamacrocyclic N-Alkylphosphines or Acid Anhydrides thereof

The procedures in Examples 1, 2 and 3 may be readily adapted by those of skill in the art of synthetic organic chemistry to any suitably stable polyazamacrocyclic compound. Suitable polyazamacrocyclic compounds include the following triaza and tetraaza macrocyclic compounds available from the Aldrich Chemical Co, Milwaukee, Wisconsin:

1,5,9-triazacyclododecane (see Figure 10);
1,4,8,12-tetraazacyclopentadecane (see Figure 11); and
1,4,8,11-tetraazacyclotetradecane (see Figure 12).

The following citations are incorporated by reference herein insofar as their pertinence for the reasons cited.

**REFERENCES**


1. E. Carafoli and J.T. Penniston, *Sci. Amer.* 253:70 (1985).

2. R.D. Grubbs and M.E. Maguire, *Magnesium* 6:113 (1983).

3. G. Charton, G. Rovira, Y. Ben-Ari and V. Leviel, *Exp Brain Res.* 58:202 (1985).

4. D. Veloso, R.W. Gwynn, M. Oskarsson, and R.L. Veech, *J. Biol Chem* 248:4811 (1973).

5. J.R. Lopez, L. Alamo, C. Caputo, J. Vergara, and R. Di Polo, *Biochim. Biophys. Acta* 804:1 (1984).

6. C. H. Fry, *Magnesium* 5:306 (1986).

7. F.J. Brinkley and A. Scarpa, *FEBS Lett* 50:82 (1975).

8. T.J. Rink, R.Y. Tsien, and T. Pozzan, *J. Cell. Biol.* 95:189 (1982).

9. B.E. Corkey, J. Duszynski, T.L. Rich, B. Matschinsky, and J.R. Williamson, *J. Biol. Chem.* 258:14294 (1986).

10. G. A. Smith, T. R. Hesketh, J.C. Metcalfe, J. Feeney, and P.G. Morris, *Proc. Natl. Acad. Sci. USA* 80:7178 (1983).

11. J.C. Metcalfe, T.R. Hesketh, and G.A. Smith, *Cell Calcium* 6:183 (1985).

12. L.A. Levy, E. Murphy, B. Raju, and R.E. London, *Biochemistry* 27:4041 (1988).

13. E. Murphy, C. Steenbergen, L.A. Levy, B. Raju, and R.E. London, *J. Biol. Chem.* 264:5622 (1989).

14. E. Marban, M. Kitakaze, H. Kusuoka, J.K. Poterfield, D.T. Yue, and V.P. Chacko, *Proc. Natl. Acad. Sci. USA* 84:6005 (1987).

15. H.M. Irving, M.C. Miles, and L.D. Petit, *Anal. Chim. Acta* 38 475 (1967).

16. M.S. Cacei and W.P. Cacheris, *Byte* 5:340 (1984).

17. (a) C.F.G.C. Geraldes, A.D. Sherry, and W.P. Cacheris, *Inorg. Chem.* 28:336 (1989). (b) S. Cortes, E. Brucher, C.F.G.C. Geraldes, and A.D. Sherry *Inorg. Chem.* 29:5 (1990)/

18. M.I. Kabachnik, T.Ya. Medved, Yu.M. Polikarpov, B.K. Scherbakov, F.I. Belskii, E.I. Matrosov, and M.P. Pasechnik, *Izv. Akad. Nauk. SSSR Ser Khim* 769 (1984).

19. R.K. Gupta, J.L. Benovic, and Z.B. Rose, *J. Biol. Chem.* 253:6472 (1978).

20. P.W. Flatman and V.L. Lew, *Nature* (London) 267:360 (1977).

21. A. Bevilacqua, R.T. Gelb, W.B. Hobard, and L.F. Zompa, *Inorg. Chem.* 26:2699 (1987).

**Claims**

**1.** A polyazamacrocyclic compound or a salt thereof, the compound having the formula:

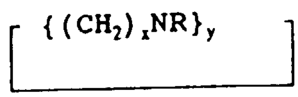

where

x is 2, 3 or a combination of p 2(s) and q 3(s) where p + q = y;
y is 3 or 4;
R is$(CH_2)_zP(=O)OR^1R^2$;
when y is 3, $R^1$ is O-alkyl and $R^2$ is attached to the oxygen atom and is alkyl or

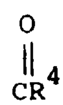

where $R^4$ is alkyl, cycloalkyl or aryl; and
z is 1 to 3;
when y is 4, $R^1$ is $OR^3$ where $R^3$ is alkyl with 2 or more carbon atoms and $R^2$ is attached to the oxygen atom
a is hydrogen, alkyl or

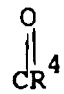

where $R^4$ is alkyl, cycloalkyl or aryl; and
z is 1 to 3.

2. A polyazamacrocyclic compound-metal complex having the formula:

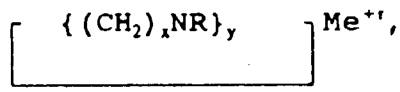

where where

x is 2, 3 or a combination of p 2(s) and q 3(s) where p + q = y;
y is 3 or 4;
R is $(CH_2)_ZP(=O)OR^1R^2$;
when y is 3, $R^1$ is O-alkyl and $R^2$ is attached to the oxygen atom and is alkyl or

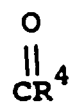

where $R^4$ is alkyl, cycloalkyl or aryl; and
z is 1 to 3; and
when y is 4, $R^1$ is $OR^3$ where
$R^3$ is alkyl with 2 or more carbon atoms and $R^2$ is attached to the oxygen atom and is hydrogen, alkyl or

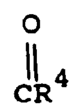

where $R^4$ is alkyl, cycloalkyl or aryl; and
z is 1 to 3;
r is 2 or 3; and
Me is a metal ion.

3. The compound of claim 1 or 2 where y is 3.

4. The compound of claim 1 or 2 where y is 4.

5. The compound of claim 1 or 2 where y is 3 and x is 2.

6. The compound of claim 1 or 2 where y is 4 and x is 2.

7. The compound of claim 1 or 2 where y is 3, p is 1 and q is 2 or p is 2 and q is 1.

8. The compound of claim 1 or 2 where y is 4, p is 1 and q is 3, p is 2 and q is 2 or p is 3 and q is 1.

9. The compound of claim 1 or 2 where z is 1.

**10.** The compound of claim 1 or 2 where alkyl is ethyl.

**11.** The complex of claim 2 where $Me^{+r}$ is $Ca^{+2}$, $Mg^{+2}$, $Zn^{+2}$ or $Gd^{+3}$.

**12.** A compound or salt thereof, the compound having the formula:

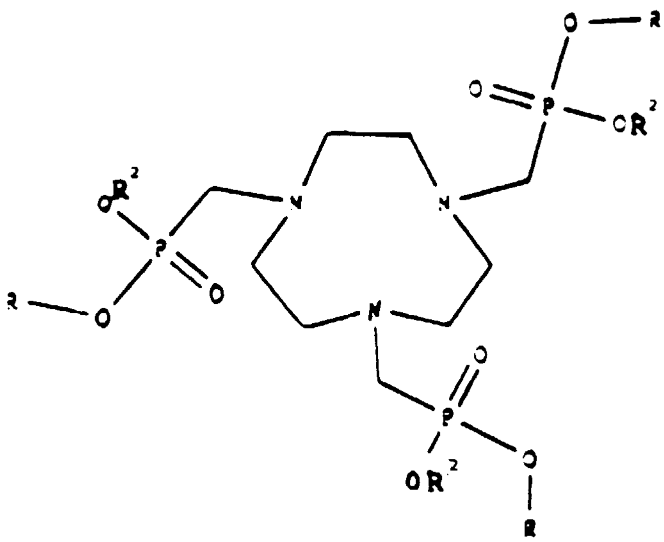

where R is $C_nH_{1+2n}$; and
$R^2$ is alkyl or

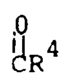

where $R^4$ is alkyl, cycloalkyl or aryl.

**13.** A compound of claim 12 where n is 1 to 20.

**14.** A method for assessing intracellular concentration of a divalent metal ion, the method comprising treating cells in vitro, under conditions facilitating intracellular localization, of a polyazamacrocyclic compound or a salt thereof, the compound having the formula

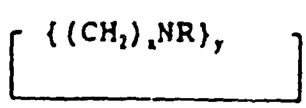

where

x is 2, 3 or a combination of p 2(s) and q 3(s) where p + q = y;
y is 3 or 4;
R is $(CH_2)_ZP(=O)OR^1R^2$;
when y is 3, $R^1$ is O alkyl and $R^2$ is attached to the oxygen atom and is alkyl or

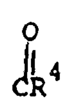

where $R^4$ is alkyl, cycloalkyl or aryl; and
z is 1 to 3 and
when y is 4, $R^1$ is $OR^3$ where $R^3$ is alkyl with 2 or more carbon atoms and $R^2$ is attached to the oxygen atom and is hydrogen, alkyl or

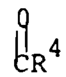

where $R^4$ is alkyl, cycloalkyl or aryl; and z is 1to 3;

and measuring changes in the $^{31}P$ NMR spectrum, said changes being proportional to intracellular divalent metal concentration.

**15.** The method of claim 14 wherein the metal is calcium, magnesium or zinc.

**16.** The method of claim 14 wherein the metal is magnesium.

**17.** The method of claim 14 wherein x is 2, y is 3 and z is 1 and alkyl is ethyl.

**18.** The method claim 14 wherein x is 2, y is 4 and z is 1 and alkyl is ethyl.

**19.** The use of a polyazamacrocyclic compound-metal complex having the formula

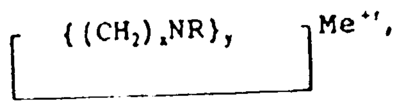

where

x is 2, 3 or a combination of p 2(s) and q 3(s) where p + q = y;
y is 3 or 4;
R is $(CH_2)_ZP(=O)OR^1R^2$;
when y is 3, $R^1$ is O alkyl and $R^2$ is attached to the oxygen atom and is alkyl or

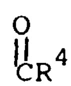

where $R^4$ is alkyl, cycloalkyl or aryl; and
z is 1 to 3;
when y is 4, $R^1$ is $OR^3$ where
$R^3$ is alkyl with 2 or more carbon atoms and $R^2$ is attached to the oxygen atom and is hydrogen, alkyl or

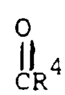

where $R^4$ is alkyl, cycloalkyl or aryl; and
z is 1 to 3;
Me is a paramagnetic metal; and
r is 2 or 3;

as a magnetic resonance image enhancement agent.

**20.** The use of claim 19 wherein the metal is a lanthanide.

**21.** The use of claim 19 wherein the metal is gadolinium and r is 3.

**Patentansprüche**

**1.** Polyazamacrocyclische Verbindung oder ein Salz davon, wobei die Verbindung die Formel

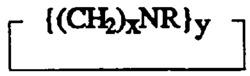

besitzt, wobei

x 2, 3 oder eine Kombination von p 2(s) und q 3(s) mit p + q = y ist;
y die Bedeutung 3 oder 4 hat;
R die Bedeutung $(CH_2)_zP(=O)OR^1R^2$ hat;
wenn y 3 ist, $R^1$ O-Alkyl bedeutet und $R^2$ an das Sauerstoffatom gebunden ist und Alkyl oder

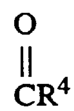

ist, wobei $R^4$ die Bedeutung Alkyl, Cycloalkyl oder Aryl hat; und
z die Bedeutung 1 bis 3 hat;
wenn y 4 ist, $R^1$ die Bedeutung $OR^3$ hat, wobei $R^3$ Alkyl mit 2 oder mehr Kohlenstoffatomen bedeutet und $R^2$ an das Sauerstoffatom gebunden ist und Wasserstoff, Alkyl oder

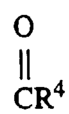

ist, wobei $R^4$ die Bedeutung Alkyl, Cycloalkyl oder Aryl hat; und
z die Bedeutung 1 bis 3 hat.

**2.** Polyazamacrocyclische Verbindung-Metall-Komplex der Formel

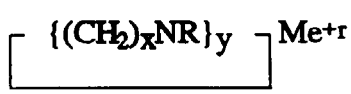

wobei

x 2, 3 oder eine Kombination von p 2(s) und q 3(s) mit p + q = y ist;
y die Bedeutung 3 oder 4 hat;
R die Bedeutung $(CH_2)_zP(=O)OR^1R^2$ hat;
wenn y 3 ist, $R^1$ O-Alkyl bedeutet und $R^2$ an das Sauerstoffatom gebunden ist und Alkyl oder

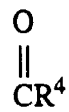

ist, wobei $R^4$ die Bedeutung Alkyl, Cycloalkyl oder Aryl hat; und
z die Bedeutung 1 bis 3 hat; und
wenn y 4 ist, $R^1$ $OR^3$ bedeutet, wobei $R^3$ Alkyl mit 2 oder mehr Kohlenstoffatomen ist und $R^2$ an das Sauerstoffatom gebunden ist und Wasserstoff, Alkyl oder

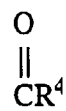

ist, wobei $R^4$ die Bedeutung Alkyl, Cycloalkyl oder Aryl hat; und
z die Bedeutung 1 bis 3 hat;
r 2 oder 3 ist; und
Me ein Metallion bedeutet.

**3.** Verbindung nach Anspruch 1 oder 2, wobei y den Wert 3 hat.

**4.** Verbindung nach Anspruch 1 oder 2, wobei y den Wert 4 hat.

**5.** Verbindung nach Anspruch 1 oder 2, wobei y den Wert 3 und x den Wert 2 haben.

**6.** Verbindung nach Anspruch 1 oder 2, wobei y den Wert 4 und x der Wert 2 haben.

**7.** Verbindung nach Anspruch 1 oder 2, wobei y den Wert 3, p den Wert 1 und q den Wert 2 oder p den Wert 2 und q den Wert 1 haben.

**8.** Verbindung nach Anspruch 1 oder 2, wobei y den Wert 4, p den Wert 1 und q den Wert 3, p den Wert 2 und q den Wert 2 oder p der Wert 3 und q den Wert 1 haben.

**9.** Verbindung nach Anspruch 1 oder 2, wobei z den Wert 1 hat.

**10.** Verbindung nach Anspruch 1 oder 2, wobei Alkyl die Bedeutung Ethyl hat.

**11.** Komplex nach Anspruch 2, wobei $Me^{+r}$ die Bedeutung $Ca^{+2}$, $Mg^{+2}$, $Zn^{+2}$ oder $Gd^{+3}$ hat.

**12.** Verbindung oder Salz davon, wobei die Verbindung die Formel

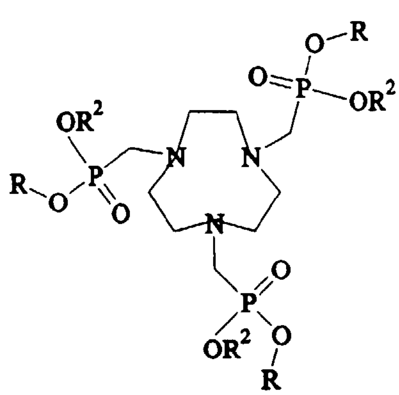

hat, wobei R die Bedeutung $C_nH_{1+2n}$ hat; und
$R^2$ die Bedeutung Alkyl oder

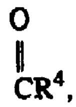

wobei $R^4$ Alkyl, Cycloalkyl oder Aryl ist.

**13.** Verbindung nach Anspruch 12, wobei n einen Wert von 1 bis 20 hat.

**14.** Verfahren zur Bestimmung der intrazellulären Konzentration eines zweiwertigen Metallions, wobei das Verfahren umfaßt die Behandlung der Zellen in vitro unter Bedingungen, die die intrazelluläre Lokalisation einer polyazamacrocyclischen Verbindung oder eines Salzes davon erleichtern, wobei die Verbindung die Formel

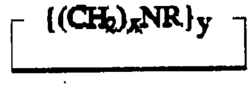

besitzt, wobei

x 2, 3 oder eine Kombination von p 2(s) und q 3(s) mit p + q = y ist;
y die Bedeutung 3 oder 4 hat;
R die Bedeutung $(CH_2)_zP(=O)OR^1R^2$ hat;
wenn y 3 ist, $R^1$ O-Alkyl bedeutet und $R^2$ an das Sauerstoffatom gebunden ist und Alkyl oder

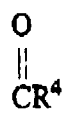

ist, wobei $R^4$ die Bedeutung Alkyl, Cycloalkyl oder Aryl hat; und
z die Bedeutung 1 bis 3 hat, und
wenn y 4 ist, $R^1$ die Bedeutung $OR^3$ hat, wobei $R^3$ Alkyl mit 2 oder mehr Kohlenstoffatomen bedeutet und $R^2$ an das Sauerstoffatom gebunden ist und Wasserstoff, Alkyl oder

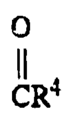

ist, wobei $R^4$ die Bedeutung Alkyl, Cycloalkyl oder Aryl hat; und
z die Bedeutung 1 bis 3 hat;

und Messung der Veränderungen im $^{31}P$ NMR-Spektrum, wobei die Änderungen der intrazellulären zweiwertigen Metallkonzentration proportional sind.

**15.** Verfahren nach Anspruch 14, wobei das Metall Calcium, Magnesium oder Zink ist.

**16.** Verfahren nach Anspruch 14, wobei das Metall Magnesium ist.

**17.** Verfahren nach Anspruch 14, wobei x den Wert 2 und y den Wert 3 hat, und z den Wert 1 hat und Alkyl oder Ethyl ist.

**18.** Verfahren nach Anspruch 14, wobei x den Wert 2 und y den Wert 4 hat, und z den Wen 1 hat und Alkyl oder Ethyl ist.

**19.** Verwendung eines polyazamacrocyclische Verbindung-Metall-Komplexes der Formel

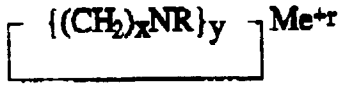

wobei

x 2, 3 oder eine Kombination von p 2(s) und q 3(s) mit p + q = y ist;
y die Bedeutung 3 oder 4 hat;
R die Bedeutung $(CH_2)_zP(=O)OR^1R^2$ hat;
wenn y 3 ist, $R^1$ O-Alkyl bedeutet und $R^2$ an das Sauerstoffatom gebunden ist und Alkyl oder

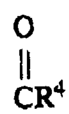

ist, wobei $R^4$ die Bedeutung Alkyl, Cycloalkyl oder Aryl hat; und
z die Bedeutung 1 bis 3 hat;
wenn y 4 ist, $R^1$ $OR^3$ bedeutet, wobei $R^3$ Alkyl mit 2 oder mehr Kohlenstoffatomen ist und $R^2$ an das Sauerstoffatom gebunden ist und Wasserstoff, Alkyl oder

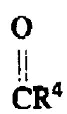

ist, wobei $R^4$ die Bedeutung Alkyl, Cycloalkyl oder Aryl hat; und
z die Bedeutung 1 bis 3 hat;
Me ein paramagnetisches Metall ist; und
r 2 oder 3 ist;

als magnetische Resonanz Bild-verstärkendes Mittel.

**20.** Verwendung nach Anspruch 19, wobei das Metall ein Lanthanide ist.

**21.** Verwendung nach Anspruch 19, wobei das Metall Gadolinium ist, und r den Wert 3 hat.

**Revendications**

**1.** Composé polyazamacrocyclique, ou un sel de celui-ci, le composé en question répondant à la formule :

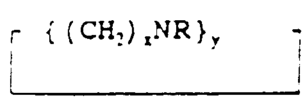

dans laquelle

x est 2, 3 ou une combinaison de p (2s) et de q 3(s), où p + q = y;

y est 3 ou 4;
R est $(CH_2)_zP(=O)OR^1R^2$;
lorsque y est 3, $R^1$ est O-alkyle et $R^2$ est attaché à l'atome d'oxygène et est alkyle ou

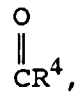

où $R^4$ est alkyle, cycloalkyle ou aryle; et
z varie de 1 à 3:
lorsque y est 4, $R^1$ est $OR^3$, où $R^3$ est alkyle avec 2 ou plus de deux atomes de carbone et $R^2$ est attaché à l'atome d'oxygène et est un atome d'hydrogène, un radical alkyle, ou

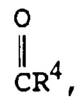

où $R^4$ est alkyle, cycloalkyle ou aryle; et
z varie de 1 à 3.

**2.** Complexe de métal d'un composé polyazamacrocyclique, répondant à la formule suivante :

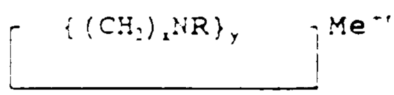

dans laquelle

x est 2, 3 ou une combinaison de p (2s) et de q 3(s), où p + q = y;
y est 3 ou 4;
R est $(CH_2)_zP(=O)OR^1R^2$;
lorsque y est 3, $R^1$ est O-alkyle et $R^2$ est attaché à l'atome d'oxygène et est alkyle ou

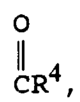

où $R^4$ est alkyle, cycloalkyle ou aryle; et
z varie de 1 à 3; et
lorsque y est 4, $R^1$ est $OR^3$, où $R^3$ est alkyle avec 2 ou plus de deux atomes de carbone et $R^2$ est attaché à l'atome d'oxygène et est un atome d'hydrogène, un radical alkyle, ou

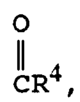

où $R^4$ est alkyle, cycloalkyle ou aryle; et
z varie de 1 à 3;
r est 2 ou 3; et
Me est un ion de métal.

**3.** Composé suivant la revendication 1 ou 2, où y est 3.

**4.** Composé suivant la revendication 1 ou 2, où y est 4.

**5.** Composé suivant la revendication 1 ou 2, où y est 3 et x est 2.

**6.** Composé suivant la revendication 1 ou 2, où y est 4 et x est 2.

**7.** Composé suivant la revendication 1 ou 2, où y est 3, p est 1 et q est 2 ou bien p est 2 et q est 1.

**8.** Composé suivant la revendication 1 ou 2, où y est 4, p est 1 et q est 3, p est 2 et q est 2, ou bien p est 3 et q est 1.

**9.** Composé suivant la revendication 1 ou 2, où z est 1.

**10.** Composé suivant la revendication 1 ou 2, où alkyle est éthyle.

**11.** Le complexe de la revendication 2, où $Me^{+r}$ est $Ca^{+2}$, $Mg^{+2}$, $Zn^{+2}$ ou $Gd^{+3}$.

**12.** Composé ou sel de celui-ci, le composé répondant à la formule :

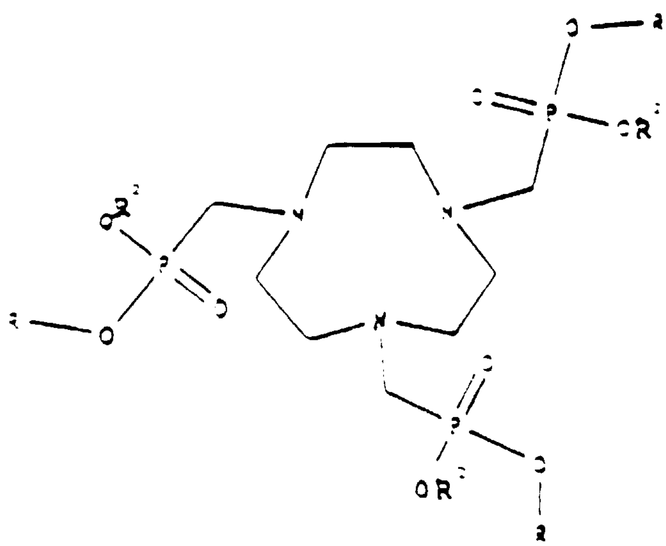

dans laquelle R est $C_nH_{1+2n}$; et
$R^2$ est alkyle ou

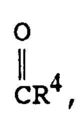

où $R^4$ est alkyle, cycloalkyle ou aryle.

**13.** Composé suivant la revendication 12, caractérisé en ce que n varie de 1 à 20.

**14.** Procédé pour vérifier la concentration intracellulaire d'un ion de métal divalent, caractérisé en ce que l'on traite des cellules in vitro, dans des conditions qui facilitent la localisation intracellulaire, par un composé polyazamacrocyclique ou un sel de celui-ci, le composé possédant la formule suivante :

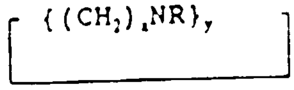

dans laquelle

x est 2, 3 ou une combinaison de p (2s) et de q 3(s), où p + q = y;
y est 3 ou 4;
R est $(CH_2)_zP(=O)OR^1R^2$;
lorsque y est 3, $R^1$ est O-alkyle et $R^2$ est attaché à l'atome d'oxygène et est alkyle ou

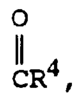

où $R^4$ est alkyle, cycloalkyle ou aryle; et
z varie de 1 à 3; et
lorsque y est 4, $R^1$ est $OR^3$, où $R^3$ est alkyle avec 2 ou plus de deux atomes de carbone et $R^2$ est attaché à l'atome d'oxygène et est un atome d'hydrogène, un radical alkyle, ou

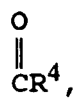

où $R^4$ est alkyle, cycloalkyle ou aryle; et
z varie de 1 à 3;
et on mesure les changements du spectre de $^{31}P$ RMN, lesdits changements étant proportionnels à la concentration en métal divalent intracellulaire.

**15.** Procédé suivant la revendication 14, caractérisé en ce que le métal est le calcium, le magnésium ou le zinc.

**16.** Procédé suivant la revendication 14, caractérisé en ce que le métal est le magnésium.

**17.** Procédé suivant la revendication 14, caractérisé en ce que x est 2, y est 3 et z est 1 et alkyle est éthyle.

**18.** Procédé suivant la revendication 14, caractérisé en ce que x est 2, y est 4 et z est 1 et alkyle est éthyle.

**19.** Utilisation d'un complexe de métal et d'un composé polyazamacrocyclique répondant à la formule :

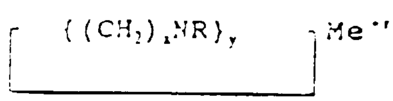

dans laquelle

x est 2, 3 ou une combinaison de p (2s) et de q 3(s), où p + q = y;
y est 3 ou 4;
R est $(CH_2)_zP(=O)OR^1R^2$;
lorsque y est 3, $R^1$ est O-alkyle et $R^2$ est attaché à l'atome d'oxygène et est alkyle ou

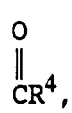

où $R^4$ est alkyle, cycloalkyle ou aryle; et
z varie de 1 à 3:
lorsque y est 4, $R^1$ est $OR^3$, où $R^3$ est alkyle avec 2 ou plus de deux atomes de carbone et $R^2$ est attaché à l'atome d'oxygène et est un atome d'hydrogène, un radical alkyle, ou

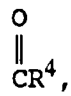

où $R^4$ est alkyle, cycloalkyle ou aryle; et
z varie de 1 à 3;
Me est un métal paramagnétique; et
r est 2 ou 3;

à titre d'agent d'amélioration de l'image obtenue par résonance magnétique.

**20.** Utilisation suivant la revendication 19, caractérisée en ce que le métal est un lanthanide.

**21.** Utilisation suivant la revendication 19, caractérisée en ce que le métal est le gadolinium et r est 3.

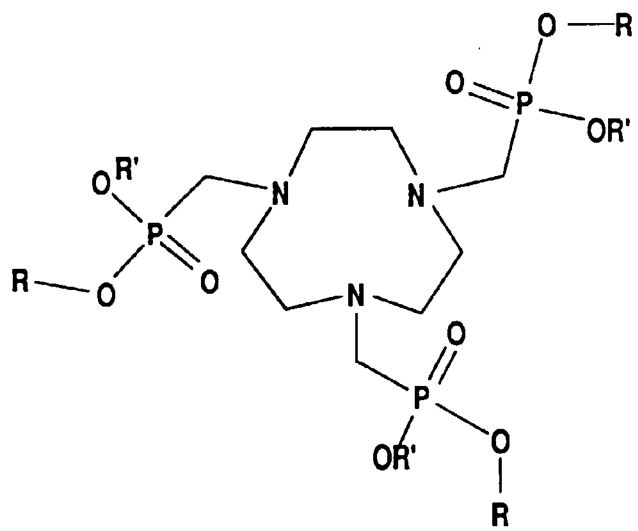

Fig. 1

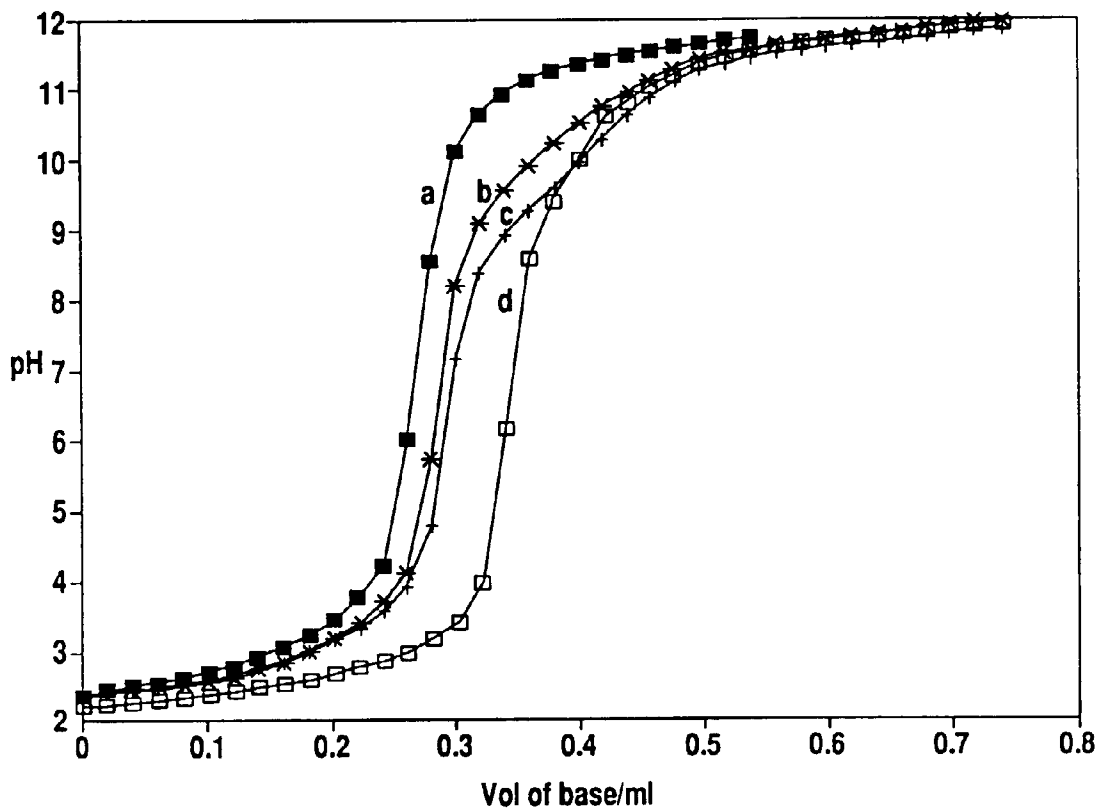


Fig. 2

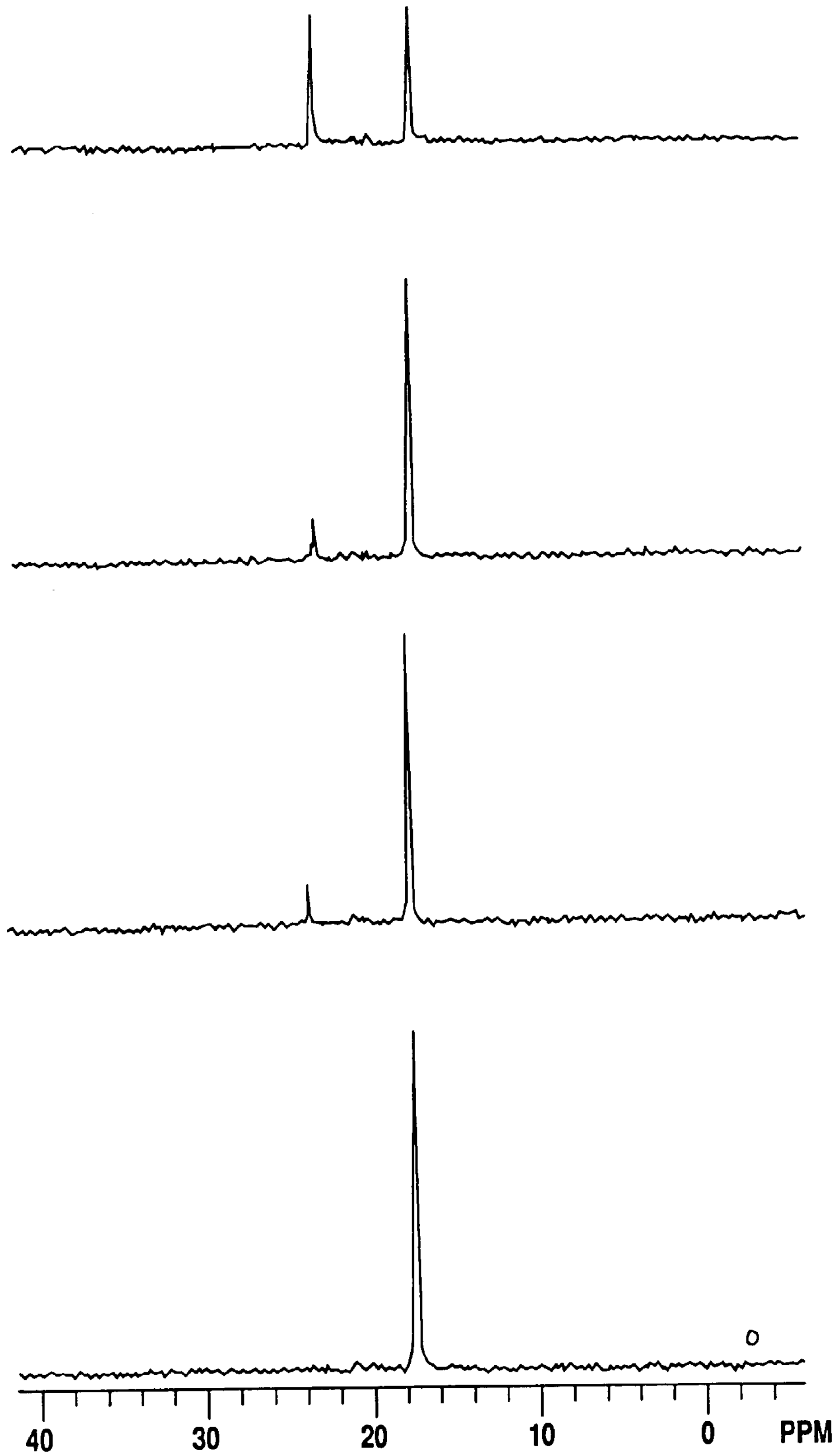


Fig. 3

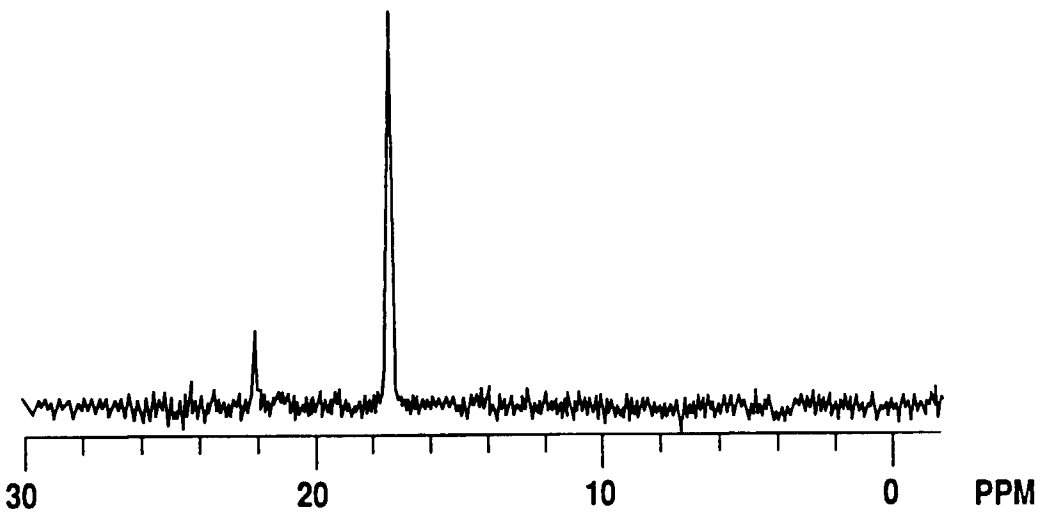


Fig. 4B

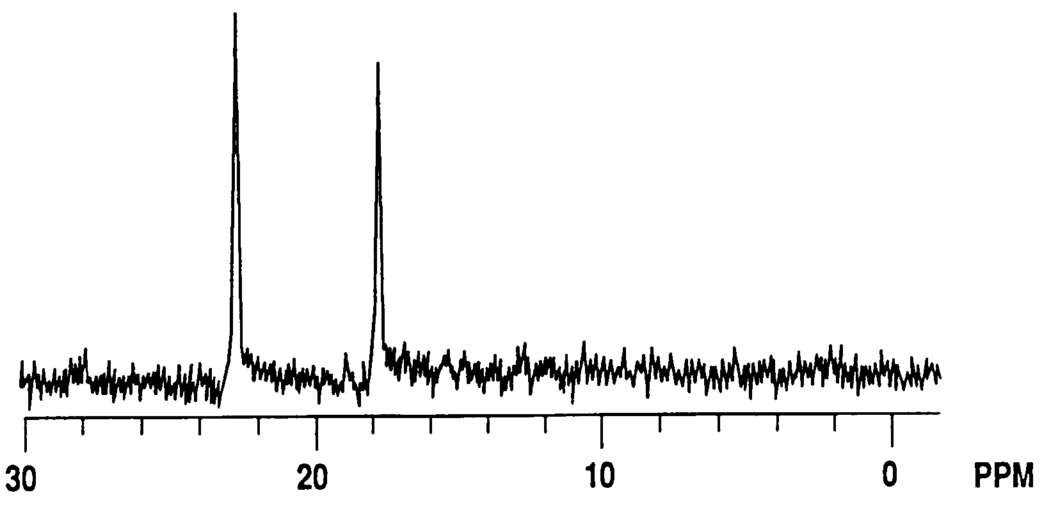


Fig. 4A

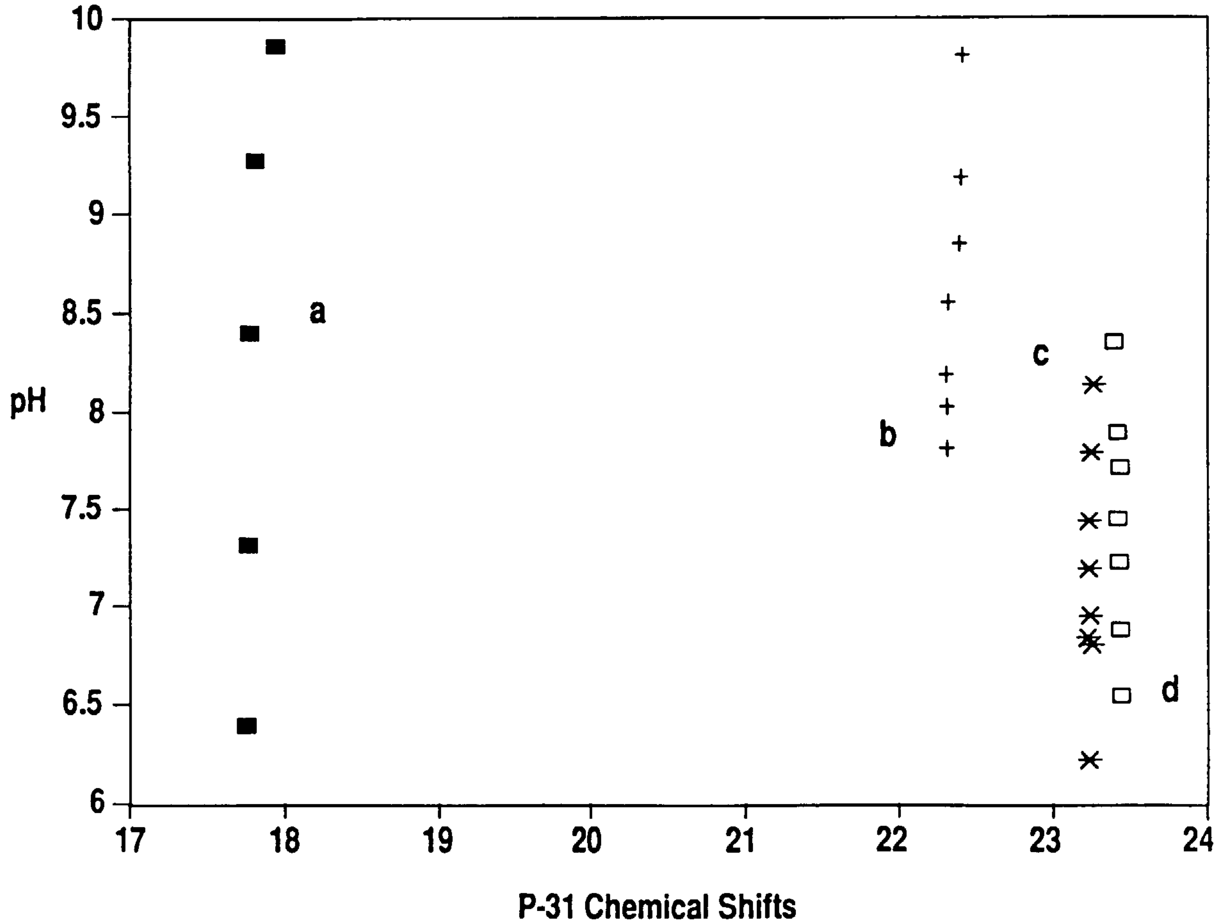
10
9.5
9
8.5
8
7.5
7
6.5
6
pH
17
18
19
20
21
22
23
24
P-31 Chemical Shifts
a
b
c
d

Fig. 5

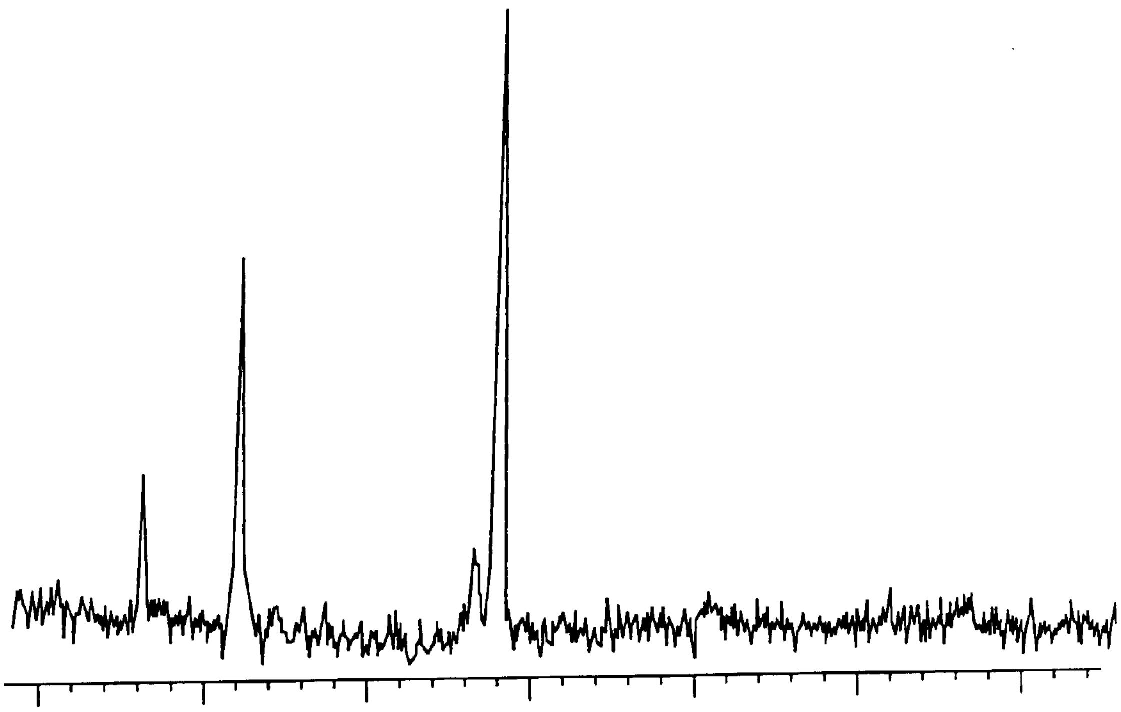

Fig. 6B

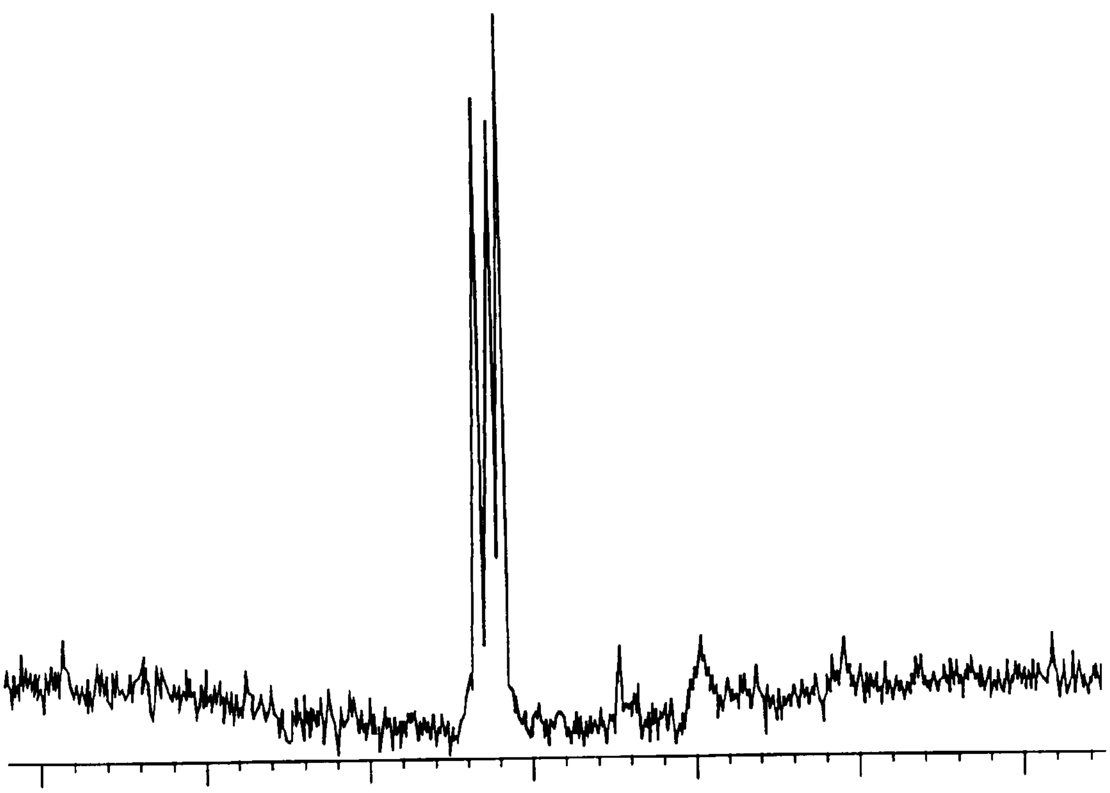

Fig. 6A

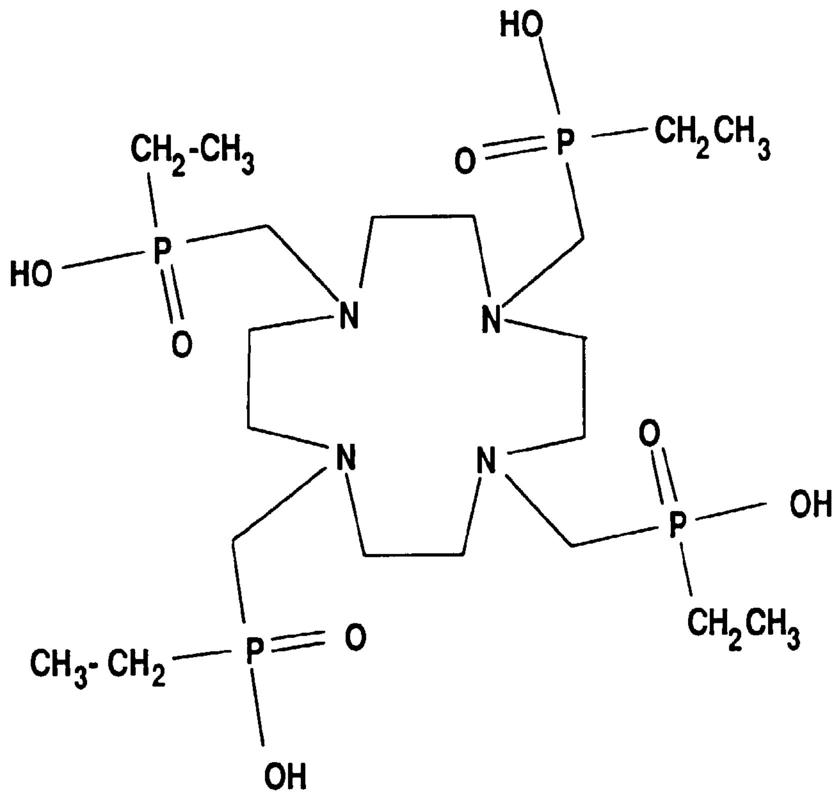

Fig. 7

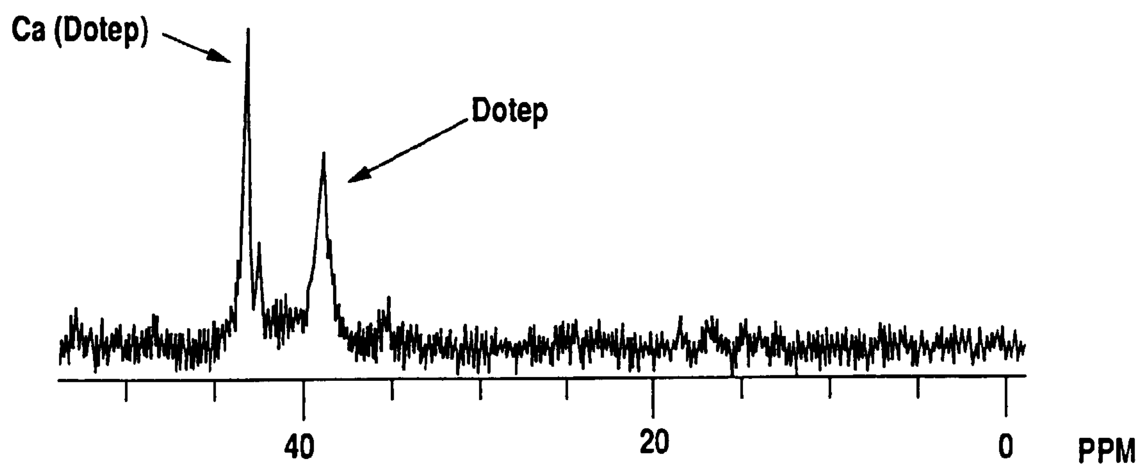


Fig. 8

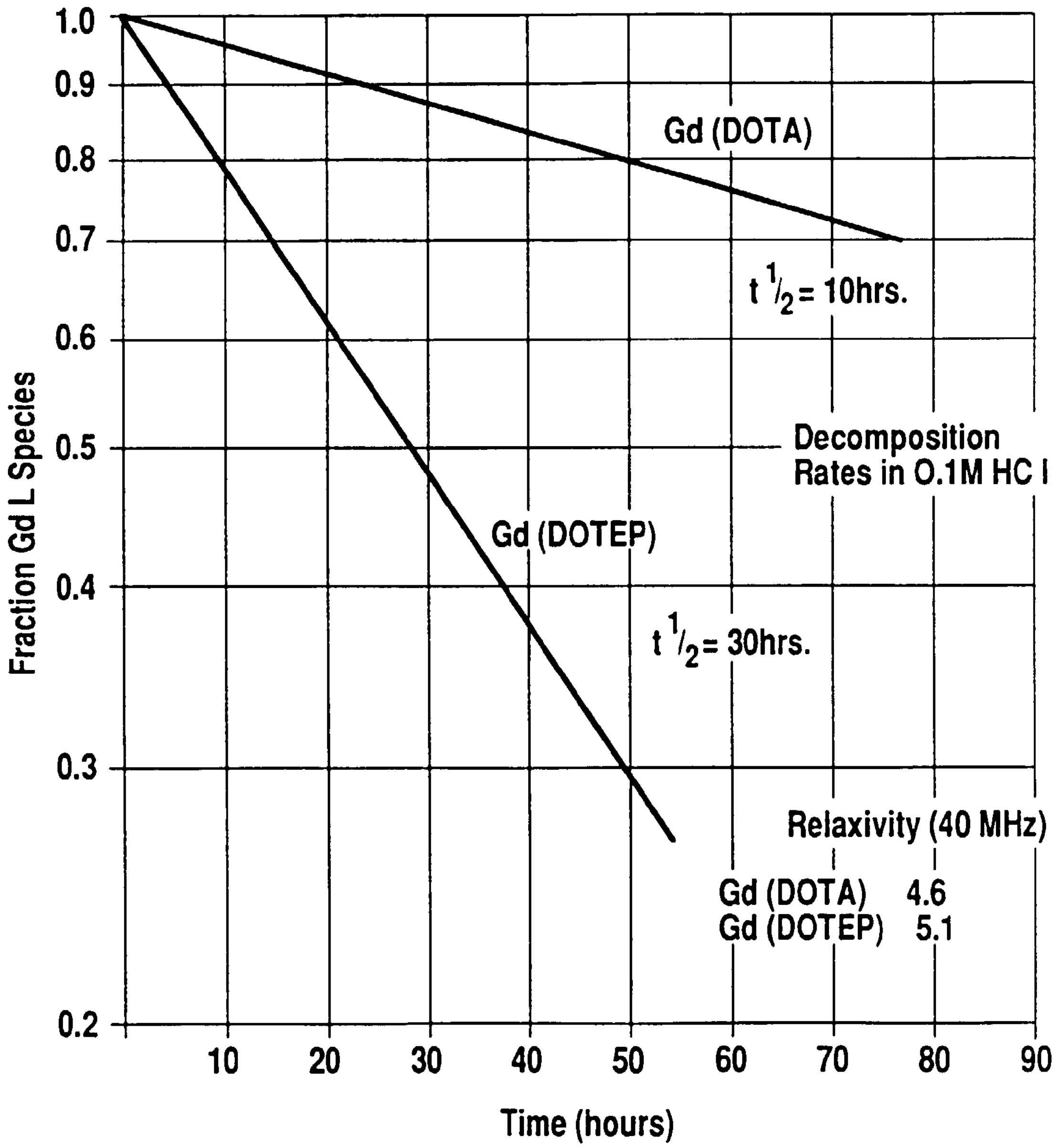
1.0
0.9
0.8
0.7
0.6
0.5
0.4
0.3
0.2
Fraction Gd L Species
10
20
30
40
50
60
70
80
90
Time (hours)
Gd (DOTA)
t 1/2 = 10hrs.
Decomposition
Rates in O.1M HC l
Gd (DOTEP)
t 1/2 = 30hrs.
Relaxivity (40 MHz)
Gd (DOTA) 4.6
Gd (DOTEP) 5.1

Fig. 9

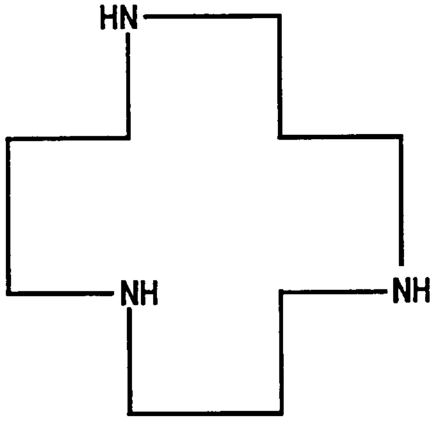

Fig. 10

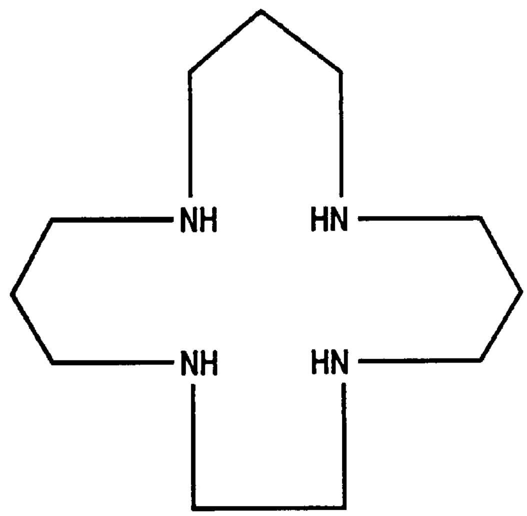

Fig. 11

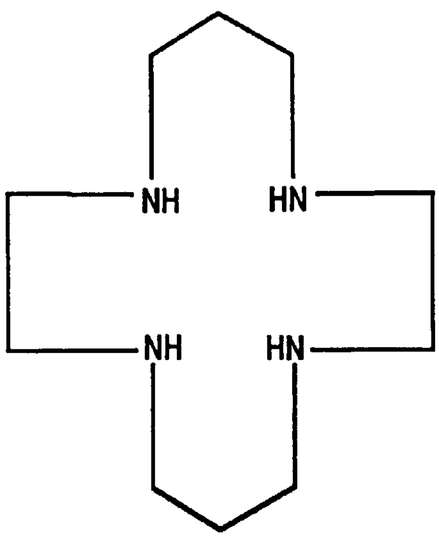

Fig. 12